# FASCICULE DE BREVET EUROPEEN

(11) **EP 0 602 056 B1**
(45) Date de publication et mention de la délivrance du brevet: **14.02.2001**
(21) Numéro de dépôt: 92916504.1
(22) Date de dépôt: 17.07.1992
(51) Int. Cl.: A61L 27/00, A61L 31/00, A61L 29/00, A61L 17/00

(54) **PROCEDE DE PROTECTION DES PROTHESES DES MATERIAUX IMPLANTABLES PROVISOIRES OU DEFINITIFS CONTRE LA COLONISATION ET L'INFECTION BACTERIENNE**
VERFAHREN ZUM SCHÜTZEN VON PROTHESEN UND PROVISORISCH ODER PERMANENT IMPLANTIERBARE MATERIALIEN GEGEN BAKTERIELLE KOLONISATION UND INFEKTION
METHOD FOR PROTECTING PROSTHESES AND TEMPORARILY OR PERMANENTLY IMPLANTABLE MATERIALS FROM BACTERIAL COLONISATION AND INFECTION

(30) Priorité: 18.07.1991 FR 9109069
(43) Date de publication de la demande: 22.06.1994
(73) Titulaire: ETESSE CARSENTI, Hélène, F-06100 Nice (FR); DELLAMONICA, Pierre, F-06100 Nice S.-Pancrace (FR)
(72) Inventeur: ETESSE CARSENTI, Hélène, F-06100 Nice (FR); DELLAMONICA, Pierre, F-06100 Nice S.-Pancrace (FR)
(74) Mandataire: Hautier, Jean-Louis
(86) Numéro de dépôt international: FR9200694
(87) Numéro de publication internationale: WO9301842

(56) Documents cités:
- EP-A- 0 141 628
- EP-A- 0 207 624
- EP-A- 0 328 421
- EP-A- 0 379 269
- WO-A-89/04674
- GB-A- 2 093 348

## Description

La présente invention concerne un procédé de protection de prothèses, implants et/ou de cathéters, de matériaux implantables provisoires ou définitifs, contre la colonisation et l'infection bactérienne, ainsi que l'équipement permettant d'obtenir cette protection.

L'imprégnation de cathéters par des quinolones a été décrite dans de nombreux documents .

Le document EP-A-0.328.421 concerne une méthode de fixation d'antibactériens connus grâce à une matrice de résine polymère, préalablement dissolue dans un solvant organique. C'est cette résine qui sera ensuite fixée sur le polymère. Ce mode de fixation est indirect.

Le document EP-A-0.207.624 concerne les méthodes de préparation d'un polymère anti-infectieux de façon directe, comprenant différentes étapes. Tout d'abord, une immersion du polymère dans une solution d'agents antimicrobiens dissolus dans un solvant organique, ensuite, l'immersion du polymère dans un solvant organique pouvant contenir des sels métalliques, enfin l'immersion du polymère dans un solvant organique contenant des antimicrobiens. On sèche après chaque immersion.

Le document EP-A-0.379.269 concerne l'incorporation de chlorhexidine dans un polymère lors de sa fabrication. Cette technique fait intervenir un chauffage ainsi qu'un ammonium quaternaire du type tridodécyl méthyle ammonium chlorure (TDMAC) ceci afin de permettre une fixation ultérieure de l'antibiotique. C'est donc une utilisation indirecte. D'autre part, le médecin qui veut utiliser ce type de technique doit plonger le polymère dans l'antimicrobien juste avant de l'utiliser car il n'y a pas de possibilité de conservation du polymère traité avec l'antibiotique.

Le document EP-A-0.141.628 concerne l'utilisation de la chlorhexidine et donc de ses fonctions cationiques pour la fixer directement sur du latex. Dans ce document, il n'est absolument pas question de fixer un antimicrobien ou un antibactérien par la suite.

Le document GB-A-2.093.348 utilise, pour fixer l'antimicrobien ou l'antibactérien, du plâtre de Paris.

Enfin, le document WO-A-8.904.674 concerne la fixation d'un antimicrobien sur un polymère biodégradable.

Contrairement à la plupart de ces documents, la technique développée utilise préalablement un biguanide qui va se fixer sur un polymère tel qu'un cathéter, une prothèse ou un fil de suture, afin de permettre ensuite, et ensuite seulement, la fixation de l'antimicrobien. Cet effet successif ne se retrouve dans aucun des documents cités ci-dessus..

D'autre part, cette successivité permet de ne pas avoir à utiliser, ni un solvant, ni une résine et d'avoir ensuite une technique beaucoup plus simple qui permet la conservation des objets chirurgicaux qui ont été traités et la stérilisation par oxyde d'éthylène, ce qui est gage de facilité pour le chirurgien qui aura, à sa disposition, un matériel stérile prêt à l'emploi.

L'adhésion de ces agents antibactériens sur les matériels médicaux étant difficile, il en résulte une protection insuffisante pour éviter les infections bactériennes consécutives à la mise en place de la prothèse.

L'imprégnation de cathéters par des biguanides est également décrite dans la demande de brevet japonais 60.036.064. Ces protections sont néanmoins insuffisantes et peuvent entraîner des problèmes toxicologiques lors du relargage des biguanides.

Malgré les recherches entreprises, les cas d'infections en présence de matériel étranger sont en très forte progression.

Les infections provoquées par l'implantation de prothèses peuvent être notamment à l'origine d'endocardites et font appel à des traitements difficiles, voire impossibles. L'échec de ces traitements conduit le plus souvent à l'ablation du matériel ; il s'ensuit un coût social important et un risque non négligeable pour le patient.

La présente invention permet de pallier aux problèmes de fixation et de toxicité de la substance antibactérienne, et conduit de ce fait à une efficacité améliorée.

Il a été trouvé un procédé de protection selon lequel la prothèse est imprégnée successivement, ce que ne propose pas les documents cités ci-dessus :
1) par une solution d'un biguanide défini par la formule générale : 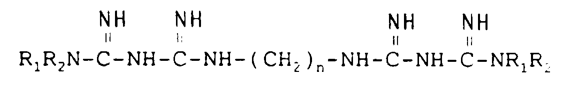 dans laquelle R₁ représente un radical alcoyle, aminoalcoyle, phényle éventuellement substitué (par un atome d'halogène ou un radical hydroxy, alcoyle, alcoyloxy ou carboxy), naphtyle ou cyano, R₂ représente un atome d'hydrogène ou un radical alcoyle et n représente un nombre entier allant de 1 à 6, étant entendu que les portions et radicaux alcoyle cités ci-dessus sont droits ou ramifiés et contiennent 1 à 4 atomes de carbone, puis
2) par une solution d'un agent antibactérien ou antibiotique autre que le biguanidine capable d'être redistribué progressivement en milieu aqueux,
   puis la prothèse ainsi imprégnée est séchée.

Les agents antibactériens convenables sont choisis parmi la classe des quinolones, des β-lactames, l'acide fusidique, la fosfomycine, la teicoplanine, l'aztréoname ou l'imipenème.

Le procédé selon l'invention permet non seulement de protéger contre la prolifération bactérienne, mais de surcroît, en utilisant le biguanide comme agent d'adhésion, permet de résoudre le double problème de la mauvaise adhérence des quinolones et d'autres antibiotiques sur les prothèses, implants et cathéters et de la toxicité des biguanides lorsqu'ils servent eux-mêmes de substance antibactérienne.

Pour arriver à ce résultat, on utilise les fonctions cationiques portées par les fonctions amines hydrogénées du biguanide et les fonctions anioniques portées par les antibiotiques; lesdites fonctions cationiques et anioniques forment une liaison ionique permettant la fixation des antibiotiques au biguanide.

Ce procédé présente l'avantage de pouvoir être appliqué à n'importe quelle sorte de prothèses utilisables en médecine et d'assurer une diffusion de l'antibiotique directement à partir du matériel implanté.

A titre d'exemple, les cathéters, implants et prothèses peuvent être choisis parmi les cathéters urinaires, des sondes, des cathéters vasculaires et intra-artériels, des prothèses valvulaires cardiaques, des prothèses artérielles, des stimulateurs cardiaques, des prothèses orthopédiques, des implants oculaires ou dentaires, des shunts de dérivation entre deux segments de l'appareil circulatoire, ainsi que des fils de suture.

Le procédé selon l'invention s'applique à des matériels de constitution variés comme des matières plastiques, des métaux ou des fils de suture.

Les solutions de biguanide employées sont avantageusement choisies parmi les solutions aqueuses ou organiques dans des solvants tels que les alcools (par exemple éthanol, méthanol), le propylèneglycol, les polyéthylèneglycols ou le glycérol.

A titre non limitatif les biguanides préférés sont la chlorhexidine : hexaméthylène bis [(p-chlorophényl)-5 biguanide] -1, 1', l'alexidine : hexaméthylène bis [(éthyl-2 hexyl)-5 biguanide]-1, 1' ou l'hexaméthylène bis [aminohexylbiguanide]-1, 1'.

Les solutions contenant l'antibiotique seront adaptées à la nature et à la classe de l'antibiotique choisi. A titre d'exemple, dans le cas des quinolones des solutions dans les alcools (éthanol par exemple) ou dans des solvants chlorés, seront particulièrement adaptées, dans le cas des β-lactames, on utilisera plus spécialement les solvants suivants : eau distillée, éthanol, méthanol.

A titre d'exemple, parmi les classes d'antibiotiques citées précédemment, les produits ci-après sont particulièrement préférés: péfloxacine, norfloxacine, sparfloxacine, énoxacine, ciprofloxacine, ofloxacine, fléroxacine, loméfloxacine, témafloxacine, amoxicilline, oxacilline, ceftriaone, cefsulodine, ceftazidime.

L'efficacité du dispositif selon l'invention a pu être mise en évidence dans les conditions suivantes :

### MATERIEL ET METHODE

### ETUDE DES MODALITES DE FIXATION DE L'ANTIBIOTIQUE

- sur des microplaques de polyvinyl (NUNC) et polystyrène (DYNATECH)
- sur des cathéters de polyurétane.

La concentration de biguanides nécessaire ainsi que la nature du solvant est étudiée (eau distillée, alcool éthylique, alcool méthylique).

Sur les microplaques, une étude de faisabilité est réalisée, puis une étude sur les conséquences de la fixation de l'antibiotique sur les propriété d'adhésion de différents germes.

Sur les cathéters, une étude de faisabilité est réalisée avec des modalités de fixation faisant varier les concentrations de l'antibiotique. Les cathéters coatés sont testés pour déterminer leur activité résiduelle en chlorhexidine, pour étudier la concentration d'antibiotique fixée, pour étudier la stabilité en fonction du temps de ce coating.

### MODALITES DU "COATING"

### a) Fixation sur des plaques de microtitration :

Les plaques sont incubées une nuit à température ambiante en présence de chlorhéxidine à concentration variable (tableau I, colonnes 2 à 11). Les plaques sont ensuite rincées à l'eau distillée ou non et séchées. L'antibiotique est alors distribué dans les cupules de la colonne 7 à la colonne 11 (tableau II). L'incubation est à nouveau réalisée pendant une nuit. Les plaques sont alors vidées, séchées à l'étuve et conservées à température ambiante, recouvertes pour éviter toute contamination par des bactéries de l'environnement. Elles sont utilisées dans un délai minimum de 8 à 60 jours après le coating, afin de vérifier la stabilité de la fixation de l'antibiotique.

Ce délai d'utilisation facilite les opérations des chirurgiens qui n'ont plus besoin, comme avec les techniques précédemment utilisées, de plonger les plaques de microtitration, ou d'autres supports, dans un agent antibiotique juste avant leur utilisation.

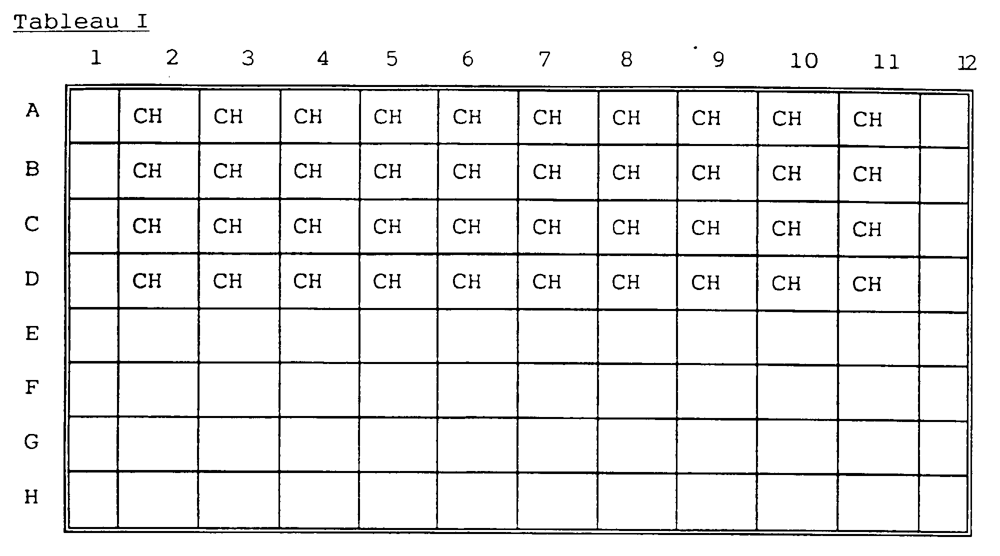

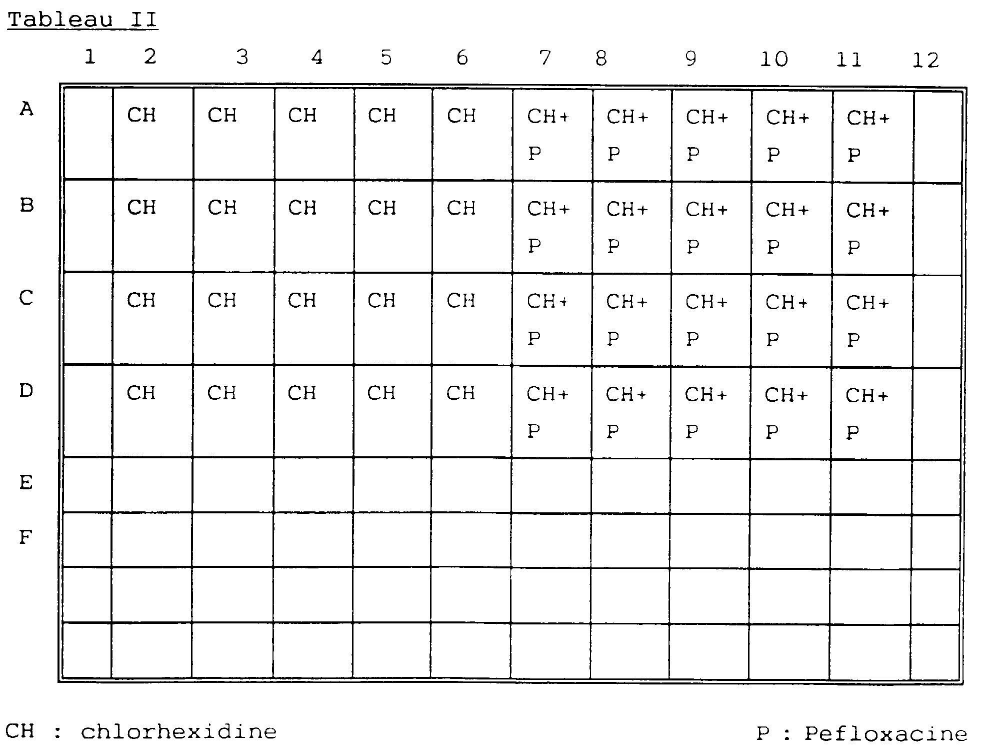

L'efficacité de la fixation de l'antibiotique est réalisée en appréciant les propriétés antibiotiques et antiadhérentielles de l'antibiotique fixé sur les cupules vis à vis des germes sensibles et/ou résistants. La méthode utilisée pour l'étude des propriétés d'adhérence est celle décrite par CHRISTENSEN G.D. et coll., J. Clin. Microbiol., 22, 996-1006 (1985). Une suspension bactérienne (inoculum de 10⁶ UFC/ml obtenu par dilution d'une culture d'une nuit) est distribuée dans les cupules de la microplaque à tester.

Après une période d'incubation de six heures à l'étuve à 37oC, les cupules sont vidées, rincées à trois reprises par du tampon phosphate pH=7 puis les bactéries restées adhérentes sont fixées à l'alcool et révélées par coloration par le cristal violet. Une lecture des densités optiques (DO) est réalisée au spectromètre (540nm).

Les résultats des cupules ayant fixé l'antibiotique sont exprimés en pourcentage d'adhésion par rapport au témoin (cupules vierges) et par rapport aux cupules chlorhexydine seules. Chaque expérience est réalisée sur la même plaque dans quatre cupules au moins et sur trois plaques différentes.

Les résultats sont exprimés par la moyenne des pourcentages d'adhérence et une étude statistique est réalisée par le test t de STUDENT.

### b) Fixation sur des cathéters :

### Etudes in vitro

Les modalités de fixation de l'antibiotique sont étudiées en faisant varier : les concentrations en chlorhéxidine et les concentrations en antibiotiques.

Une étude de stabilité des cathéters en fonction du temps est réalisée par mesure de l'activité antibiotique des cathéters conservés pendant deux mois. L'activité antibiotique est appréciée in vitro par la méthode de KIRBY-BAUER. Les morceaux de cathéters sont implantés verticalement dans une gélose inoculée par inondation avec un germe choisi (Staphylococcus aureus, Escherichia coli, Micrococcus lutea, Enterobacter cloacae, Serratia marcescens...) Après incubation, une nuit à 37oC, les diamètres d'inhibition sont mesurés, comparés à ceux des cathéters non coatés et des cathéters témoins chlorhéxidine.

Il a ainsi été démontré en faisant varier les concentrations en antibiotique (par exemple la péfloxacine), que la fixation est dose-dépendante.

### ETUDE DE RELARGAGE DE L'ANTIBIOTIQUB

- Epuisement de l'antibiotique dans une solution tampon qui est renouvelée pour connaître la libération de l'antibiotique en cas de renouvellement liquidien.
- Etude in vivo

Des morceaux de cathéters coatés avec de la péfloxacine sont implantés en intrapéritonéal selon un modèle d'infection sur cathéter. Après mise en place des cathéters, une infection est provoquée par injection d'un inoculum de Staphylococcus aureus variable selon les expériences. Cet inoculum est injecté soit 24 heures après la mise en place des cathéters, soit juste après cette implantation. Après 24 et 48 heures, les souris sont sacrifiées, les cathéters sont prélevés, traités par sonication, trypsine pour décrocher les bactéries adhérentes et cultivés sur gélose pour numérer leur contamination bactérienne. Des prélèvements concommittants sont réalisés : foie, rate et sang. Ces expériences sont réalisées par rapport à des lots témoins de souris sur lesquelles sont implantés des cathéters non fixés avec l'antibiotique.

Dans les liquides d'élution des cathéters, sont réalisés les dosages de l'antibiotique présent ainsi que sur les cathéters prélevés ; l'antibiotique résiduel est dosé par implantation directe du cathéter dans la gélose.

### c) Etude de fixation sur des implants métalliques :

Des morceaux métalliques (d'acier ou d'alliage de titane constituant des exemples de matériaux utilisés pour des prothèses métalliques) sont fixés avec la péfloxacine dans les mêmes conditions, puis testés par implantation des échantillons dans la gélose.

### d) Etudes de la fixation sur des fils de suture :

Après fixation selon les mêmes procédés, les fils séchés et conservés pendant 15 jours à 2 mois sont testés pour leur activité antibiotique sur des géloses ensemencées avec des germes divers et ce par rapport à des fils témoins non coatés ou ayant fixé seulement la chlorhexidine.

### RESULTATS

### I - RESULTATS AVEC LES PLAQUES DE MICROTITRATION

### Fixation de la péfloxacine

Les plaques sont dans un premier temps incubées une nuit à température ambiante avec la chlorhexidine (CH) aux concentrations de 0,2%, 1%, 2%, 10% en eau distillée, alcool éthylique et alcool méthylique. Certaines sont lavées et séchées, d'autres simplement lavées avant la distribution sur la moitié de la plaque de la péfloxacine à concentration variable.

Les résultats montrant l'influence du lavage des plaques après le temps de fixation de CH et avant l'antibiotique sur l'activité antiadhérentielle vis-à-vis d'acinetobacter montrent que la fixation est plus efficace si un lavage n'est pas effectué avant l'imprégnation par l'antibiotique. Des concentrations variables de chlorhexidine sont étudiées. Les résultats de l'activité antiadhérentielle de la péfloxacine, sur des germes sensibles et sur des germes résistants montrent que l'activité la plus importante est obtenue avec une concentration en chlorhexidine de 10%.

### II - RELARGAGE DE LA PEFLOXACINE FIXEE SUR CATHETER

Un cathéter imprégné par une solution contenant 10% de chlorhexidine et par une solution à 2 g/l de péfloxacine est placé dans 1 ml de tampon. On mesure la concentration en péfloxacine dans le tampon après 1 et 24 heures d'incubation à 37oC : 2,4 et 2,6mg/l respectivement. Le tampon initial est enlevé puis un dosage est effectué après incubation à 37oC une nuit. Le taux résiduel est alors de 0.3mg/l. Cette expérience permet de montrer qu'il est possible de relarguer l'antibiotique dans le milieu à partir du cathéter. Ce relargage est effectif dès 1 à 24 heures.

### III - ETUDE IN VIVO

Les résultats montrent que si l'inoculum bactérien est injecté 24 heures après mise en place des cathéters coatés, aucune protection significative n'est obtenue. Par contre, si l'inoculum est injecté le même jour que la mise en place des cathéters (ce qui correspond au modèle d'infection en chirurgie humaine), une différence significative est observée sur les moyennes en logarithme UFC/ml sur les cathéters avec un inoculum de 10⁶ UFC/ml. Cette différence est plus significative si les cathéters sont prélevés au temps 48 heures. De même, avec un inoculum bactérien de 10⁵ UFC/ml, si la protection assurée par les cathéters coatés est non significative après 24 heures, elle est significative après 48 heures. Sur les cathéters péfloxacine infectés, la colonisation bactérienne est également significativement diminuée par rapport aux cathéters témoins contaminés.

### IV - ETUDE SUR DES IMPLANTS METALLIQUES

Dans les conditions décrites précédemment une fixation de la péfloxacine est obtenue après traitement par immersion dans une solution de chlorhexidine à 10% d'alliages de titane et de fragments en acier inoxydable.

Les résultats montrent un accroissement du diamètre d'inhibition par rapport au témoin non imprégné, pour tous les germes étudiés.

D'une manière générale, le procédé de protection selon l'invention est mis en oeuvre par trempage de la prothèse, de l'implant ou du cathéter dans une solution contenant de 20 à 100g/l de biguanide, éventuellement lavage, trempage dans une solution contenant 1 à 100g/l d'antibiotique, puis éventuellement lavage, et séchage.

La durée du trempage n'est pas un facteur indispensable à la réalisation de l'invention. Cependant, il est avantageux d'effectuer un trempage pendant 1 à 24 heures dans chacune des solutions.

Les prothèses et/ou les cathéters imprégnés selon le procédé de la présente invention sont capables de relarguer progressivement la substance antibactérienne dans le milieu d'implantation pendant au moins 24 à 48 heures, et ainsi de conduire à la prévention de la contamination bactérienne des matériels utilisés.

La présente invention concerne également le dispositif de protection des prothèses, implants et/ou cathéters obtenu selon le procédé décrit précédemment et capable de relarguer progressivement la substance antibactérienne ou antibiotique autre que la biguanidine fixée à la surface.

L'intérêt de ce dispositif est considérable du fait de son efficacité et de son absence de toxicité puisque le biguanide n'est pas ou très peu relargué dans le milieu d'implantation.

Selon la présente invention, l'imprégnation des prothèses, des implants et/ou des cathéters peut être effectuée indifféremment au préalable, par exemple consécutivement à la fabrication de la prothèse ou au moment de l'emploi.

La présente invention concerne également un équipement permettant de procéder facilement à l'imprégnation du cathéter, de l'implant ou de la prothèse.

Un tel équipement est constitué :
a) d'une solution contenant 20 à 100g/l du biguanide de formule générale (I) par exemple appelée solution No 1 et destinée à la première imprégnation et
b) d'une solution contenant 1 à 100 g/l de la substance antimicrobienne ou antibiotique autre que la biguanidine par exemple appelée solution No 2 et destinée à la seconde imprégnation.

La mise en oeuvre du procédé selon l'invention est ainsi facilitée dans les cas où les prothèses et/ou cathéters sont fournis sans dispositif de protection antibactérienne.

Alternativement, par exemple dans le cas de substances pouvant présenter des problèmes de stabilité en solution pendant un temps prolongé, il est bien entendu qu'un tel équipement peut aussi comprendre, à la place du flacon de solution prête à l'emploi, un flacon de solvant et la quantitié convenable de la substance qui sera dissolue extemporanément par l'utilisateur au moment de son emploi.

L'imprégnation par la solution du biguanide peut également être réalisée dès la fabrication du matériel à implanter, la deuxième imprégnation pouvant être envisagée au moment de l'emploi, par exemple dans le cas d'un antibiotique peu stable.

## Revendications

1. Procédé de protection de prothèses, implants et/ou cathéters, de matériaux implantables provisoires ou définitifs, contre la colonisation et l'infection bactérienne dans lequel on applique au matériel à protéger, une solution d'un agent antibactérien ou antibiotique capable d'être redistribué progressivement en milieu aqueux, caractérisé en ce que pour, assurer l'adhésion de l'agent anti-bactérien au matériel à protéger, on imprègne ledit matériel successivement :
a) par une solution dans un solvant acqueux ou organique d'un biguanide de formule générale : 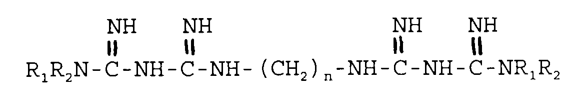 dans laquelle R₁ représente un radical alcoyle, aminoalcoyle, phényle éventuellement substitué (par un atome d'halogène ou un radical hydroxy, alcoyle, alcoyloxy ou carboxy), naphtyle ou cyano, R₂ représente un atome d'hydrogène ou un radical alcoyle et n est un nombre entier allant de 1 à 6, étant entendu que les portions et radicaux alcoyle sont droits ou ramifiés et contiennent 1 à 4 atomes de carbones, puis
b) par une solution d'un agent antibactérien ou antibiotique autre que la biguanidine capable d'être redistribué progressivement en milieu aqueux,
puis on sèche le matériel ainsi imprégné, et
que le matériel à protéger est séché entre l'imprégnation par la solution d'un biguanide et l'imprégnation par la solution d'un agent antibactérien ou antibiotique.

2. Procédé selon la revendication 1, caractérisé en ce que le matériel à protéger, après imprégnation par la solution d'un biguanide, n'est pas lavé préalablement à l'imprégnation par la solution d'un agent antibactérien ou antibiotique.

3. Procédé selon l'une quelconque des revendications 1 ou 2, caractérisé en ce que le biguanide présente des fonctions cationiques et que l'antibiotique ou l'antibactérien présente des fonctions anioniques ; lesdites fonctions cationiques et anioniques, coopérant ensemble pour fixer les antibiotiques ou les antibactériens au biguanide.

4. Procédé selon l'une quelconque des revendications 1, 2 ou 3, caractérisé en ce que le biguanide est la chlorhexidine, l'alexidine ou l'hexaméthylène bis [aminohexyl biguanide] - 1, 1'.

5. Procédé selon l'une quelconque des revendications 1, 2 ou 3, caractérisé en ce que l'agent antibiotique est choisi parmi les quinolones, les β-lactames, l'acide fusidique, la fosfomycine, la teicoplanine, l'aztréoname ou l'imipenème.

6. Procédé selon l'une quelconque des revendications 1, 2, 3 ou 5, caractérisé en ce que l'agent antibiotique est la péfloxacine.

7. Procédé selon l'une quelconque des revendications 1, 2, 3 ou 4, caractérisé en ce que l'on utilise une solution contenant 20 à 100g/l de biguanide.

8. Procédé selon l'une quelconque des revendications 1, 2, 3, 5 ou 6, caractérisé en ce que l'on utilise une solution contenant de 1 à 100 g/l d'agent antibactérien ou antibiotique.

9. Dispositif de protection des prothèses implants et/ou cathéters, capable de redistribuer progressivement en milieu aqueux une substance antibactérienne ou antibiotique fixées à sa surface, caractérisé en ce qu'il est obtenu selon le procédé de la revendication 1.

10. Equipement pour l'imprégnation de prothèses, d'implants et/ou de cathéters avant leur emploi, de manière à permettre la redistribution progressive d'une substance antibactérienne ou antibiotique autre que la biguanidine dans le milieu d'implantation, caractérisé en ce qu'il est constitué
a) d'une solution contenant 20 à 100g/l d'un biguanide selon la revendication 1 et
b) d'une solution contenant 1 à 100 g/l d'une substance antibactérienne ou antibiotique autre que la biguanidine telle que définie dans la revendication 1, ou alternativement constitué des doses de produits et de solvants nécessaires à préparer ces solutions au moment de leur emploi.

## Claims

1. Method for protecting prostheses, implants and/or catheters and temporarily or permanently implantable materials from bacterial colonisation and infection in which is applied, to the material to be protected, a solution of an antibacterial or antibiotic agent capable of being redistributed gradually in aqueous medium, characterised in that, to ensure the adhesion of the antibacterial agent to the material to be protected, the said material is impregnated successively:
a) with a solution in a aqueous or organic solvent of a biguanide of general formula: 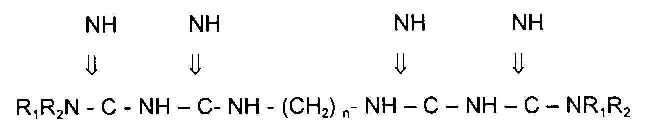 in which R₁ represents an alkyl, alkylol-amide, phenyl possibly substituted (by an atom of halogen or a hydroxy, alkyl, alcoyloxy or carboxy radical), naphtyl or cyano radical, R₂ represents an atom of hydrogen or an alkyl radical and n is an integer from 1 to 6, it being understood that the alkyl radicals and portions are straight or ramified and contain 1 to 4 atoms of carbons, and then
b) by a solution of an antibacterial or antibiotic agent other than biguanidine capable of being redistributed gradually in aqueous medium,
the material thus impregnated is then dried, and
that the material to be protected is dried between impregnation by the biguanide solution and impregnation by the antibacterial or antibiotic agent solution.

2. Method according to claim 1, characterised in that the material to be protected, after impregnation by the biguanide solution, is not washed prior to impregnation by the antibacterial or antibiotic agent solution.

3. Method according to any one of claims 1 or 2, characterised in that the biguanide displays cationic functions and that the antibiotic or antibacterial agent displays anionic functions; the said cationic and anionic functions, cooperating together to fix the antibiotic or antibacterial agents to the biguanide.

4. Method according to any one of claims 1, 2 or 3, characterised in that the biguanide is chlorhexidine, alexidine or hexamethylene B [biguanide aminohexyl] - 1, 1'.

5. Method according to any one of claims 1, 2 or 3, characterised in that the antibiotic agent is selected from among quinolones, β-lactams, fusidic acid, fosfomycin, teicoplanin, aztreoname or imipeneme.

6. Method according to any one of claims 1, 2, 3 or 5, characterised in that the antibiotic agent is pefloxacin.

7. Method according to any one of claims 1, 2, 3 or 4, characterised in that a solution containing 20 to 100 g/l of biguanide is used.

8. Method according to any one of claims 1, 2, 3, 5 or 6, characterised in that a solution containing 1 to 100 g/l of antibacterial or antibiotic agent is used.

9. Device for protecting prostheses, implants and/or catheters, capable of redistributing gradually in aqueous medium an antibacterial or antibiotic substance fixed to its surface, characterised in that it is obtained according to the method of claim 1.

10. Equipment for impregnating prostheses, implants and/or catheters before their use, in order to allow the gradual redistribution of an antibacterial or antibiotic substance other than biguanidine in the implant medium, characterised in that it is composed
a) of a solution containing 20 to 100 g/l of a biguanide according to claim 1 and
b) of a solution containing 1 to 100 g/l of an antibacterial or antibiotic substance other than biguanidine as defined in claim 1, or alternatively composed of the doses of products and solvents necessary to prepare these solutions at the time of use.

## Patentansprüche

1. Verfahren zum Schützen von Prothesen und provisorisch oder permanent implantierbaren Materialien gegen bakterielle Kolonisation und Infektion bei welchem an dem zu schützenden Material eine Lösung eines antibakteriellen bzw. antibiotischen Mittels appliziert wird, die sich fortschreitend in wasserhaltiger Umgebung verteilen kann, dadurch gekennzeichnet, daß zur Gewährleistung der Adhäsion des antibakteriellen Mittels an dem zu schützenden Material das besagte Material nacheinander imprägniert wird mit:
a) einer Lösung in einem wässerigen oder organischen Lösungsmittel eines Biguanids mit der allgemeinen Formel: 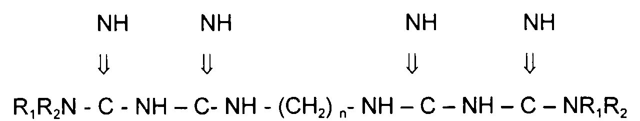 in welcher R₁ ein Alkyl-, Aminoalkyl-, eventuell (durch ein Halogenatom oder ein Hydroxy-, Alkyl-, Alkyloxy oder Carboxy-Radikal) substituiertes Phenyl-, Naphtyl- oder Cyan-Radikal darstellt, R₂ ein Wasserstoffatom oder ein Alkylradikal darstellt und eine ganze Zahl von 1 bis 6 ist, wobei vorausgesetzt ist, daß die Anteile und Alkylradikale gerade oder verzweigt sind und 1 bis 4 Kohlenstoffatome enthalten, dann
b) einer Lösung eines antibakteriellen oder antibiotischen Mittels, außer Biguanidin, die sich fortschreitend in wasserhaltiger Umgebung verteilen kann,
und anschließend das so imprägnierte Material getrocknet wird und
daß das zu schützende Material zwischen der Imprägnierung mit der Lösung eines Biguanids und der Imprägnierung mit der Lösung eines antibakteriellen oder antibiotischen Mittels getrocknet ist.

2. Verfahren gemäß Anspruch 1, dadurch gekennzeichnet, daß das zu schützende Material nach Imprägnierung mit der Lösung eines Biguanids nicht vor der Imprägnierung mit einer Lösung eines antibakteriellen oder antibiotischen Mittels gewaschen wird.

3. Verfahren gemäß irgendeinem der Ansprüche 1 oder 2, dadurch gekennzeichnet, daß das Biguanid basische Funktionen ausweist und das antibiotische oder antibakterielle Mittel anionische Funktionen aufweist; wobei die basische und anionische Funktionen zusammenwirken, um die antibiotische oder antibakterielle Mittel bzw. das Biguanid zu fixieren.

4. Verfahren gemäß irgendeinem der Ansprüche 1, 2 oder 3, dadurch gekennzeichnet, daß das Biguanid Chlorhexidin, Alexidin oder Hexamethylen a [Biguanid-Aminohexyl]-1,1' ist.

5. Verfahren gemäß irgendeinem der Ansprüche 1, 2 oder 3, dadurch gekennzeichnet, daß das antibiotische Mittel unter den Chinolonen, den β-Laktamen, der Fusidsäure, dem Fosfomyzin, dem Teicoplanin, dem Aztreonam oder dem Imipenem gewählt wird.

6. Verfahren gemäß irgendeinem der Ansprüche 1, 2, 3 oder 5, dadurch gekennzeichnet, daß das antibiotische Mittel Pefloxazin ist.

7. Verfahren gemäß irgendeinem der Ansprüche 1, 2, 3 oder 4, dadurch gekennzeichnet, daß eine Lösung benutzt wird, die 20 bis 100 g/l Biguanid enthält.

8. Verfahren gemäß irgendeinem der Ansprüche 1, 2, 3, 5 oder 6, dadurch gekennzeichnet, daß eine Lösung benutzt wird, die 1 bis 100 g/l antibakterielles oder antibiotisches Mittel enthält.

9. Einrichtung zum Schützen von implantierbaren Prothesen und/oder Kathetern, die fortschreitend in wasserhaltiger Umgebung eine antibakterielle oder antibiotische Substanz an seiner Oberfläche verteilen kann, dadurch gekennzeichnet, daß sie gemäß dem Verfahren nach Anspruch 1 erwirkt wird.

10. Ausstattung für die Imprägnierung von Prothesen, Implanten und/oder Kathetern vor ihrer Benutzung, auf eine Weise die die fortschreitende Verteilung einer antibakteriellen oder antibiotischen Substanz, außer Biguanidin, in dem Milieu der Implantation ermöglicht, dadurch gekennzeichnet, daß sie besteht aus:
a) einer Lösung mit 20 bis 100 g/l eines Biguanids gemäß Anspruch 1 und
b) einer Lösung mit 1 bis 100 g/l einer antibakteriellen oder antibiotischen Substanz, außer Biguanidin, wie in Anspruch 1 definiert, oder alternativ besteht aus den gemessenen Mengen von Produkten und Lösungsmitteln, die erforderlich sind, um diese Lösungen im Moment ihrer Anwendung herzustellen.
